# EUROPEAN PATENT SPECIFICATION

(11) **EP 4 147 249 B1**
(45) Date of publication and mention of the grant of the patent: **06.05.2026**
(21) Application number: 21731333.7
(22) Date of filing: 06.05.2021
(51) Int. Cl.: G16H 50/20, G16H 50/80

(54) **PATHOGEN TESTING**
PATHOGENTESTS
TEST DE PATHOGÈNES

(30) Priority: 06.05.2020 US 202063021059 P; 08.05.2020 US 202063022202 P
(43) Date of publication of application: 15.03.2023
(73) Proprietor: Battelle Memorial Institute, Columbus, OH 43201 (US)
(72) Inventor: SPURBECK, Rachel R., Columbus, OH 43201 (US); JOHNSON, Christopher, Columbus, OH 43201 (US); LILLY, Jacob, Columbus, OH 43201 (US); SCHUETTER, Jared M., Columbus, OH 43201 (US); WILSON, Patrick H., Columbus, OH 43201 (US)
(74) Representative: Keltie LLP
(86) International application number: PCT/US2021/031132
(87) International publication number: WO 2021/226361

(56) References cited:
- FARKAS KATA ET AL: "Seasonal and spatial dynamics of enteric viruses in wastewater and in riverine and estuarine receiving waters", SCIENCE OF THE TOTAL ENVIRONMENT, vol. 634, 1 September 2018 (2018-09-01), AMSTERDAM, NL, pages 1174 - 1183, XP055825973, ISSN: 0048-9697, Retrieved from the Internet <URL:https://www.sciencedirect.com/science/article/pii/S0048969718311951/pdfft?md5=28c8c14a8f7b34d8816e9ffdad3eb8d1&pid=1-s2.0-S0048969718311951-main.pdf> [retrieved on 20210720], DOI: 10.1016/j.scitotenv.2018.04.038
- BROUWER ANDREW F. ET AL: "Epidemiology of the silent polio outbreak in Rahat, Israel, based on modeling of environmental surveillance data | PNAS", 18 October 2018 (2018-10-18), pages 1 - 29, XP055825972, Retrieved from the Internet <URL:https://www.pnas.org/content/115/45/E10625> [retrieved on 20210720]
- BIOBOT ANALYTICS: "Testing for COVID-19 beyond the clinic: using wastewater epidemiology to proactively detect outbreaks | by Biobot Analytics | Medium", 13 March 2020 (2020-03-13), pages 1 - 6, XP055825963, Retrieved from the Internet <URL:https://biobotanalytics.medium.com/testing-for-covid-19-beyond-the-clinic-using-wastewater-epidemiology-to-proactively-detect-ede0cbf81e49> [retrieved on 20210720]
- ANONYMOUS: "Covid19 sewage - Press Office - Newcastle University", 23 April 2020 (2020-04-23), pages 1 - 3, XP055825959, Retrieved from the Internet <URL:https://www.ncl.ac.uk/press/articles/latest/2020/04/covid19sewage/> [retrieved on 20210720]
- THOMAS MACAULAY: "Startup will map the coronavirus spread by looking at poo", 20 March 2020 (2020-03-20), pages 1 - 4, XP055825962, Retrieved from the Internet <URL:https://thenextweb.com/news/this-startup-will-map-the-coronavirus-spread-by-looking-at-your-poo> [retrieved on 20210720]

## Description

### TECHNICAL FIELD

Various aspects of the present disclosure relate generally to diagnostics and in particular, to diagnostics that can be used for pathogen testing.

The monitoring of particulate matter has received an increasing amount of attention in recent years because of the potential impact of particulates on human health. As an illustration, it may be desirable to detect the presence of particulates such as naturally occurring or artificially produced pathogens, allergens, bacteria, viruses, fungi biological or chemical agents, etc., on persons, or in an particular environments, including for example, in water supplies.

PARKAS KATA ET AL: "Seasonal and spatial dynamics of enteric viruses in wastewater and in riverine and estuarine receiving waters", SCIENCE OF THE TOTAL ENVIRONMENT , vol. 634 1 September 2018 (2018-09-01), pages 1174-1183, XP055825973, AMSTERDAM, NL ISSN: 0048-9697, DOI: 10.1016/j.scitotenv.2018.04.038 relates to detection and determination of decay of enteric viruses in wastewater.

Brouwer Andrew F. ET AL: "Epidemiology of the silent polio outbreak in Rahat, Israel, based on modeling of environmental surveillance data | PNAS", , 18 October 2018 (2018-10-18), pages 1-29, XP055825972, relates to a modeling framework for environmentally mediated infectious disease.

Biobot Analytics: "Testing for COVID-19 beyond the clinic: using wastewater epidemiology to proactively detect outbreaks | by Biobot Analytics | Medium", , 13 March 2020 (2020-03-13), pages 1-6, XP055825963, relates to the detection of viral outbreaks by monitoring wastewater.

Anonymous: "Covid19 sewage - Press Office - Newcastle University", , 23 April 2020 (2020-04-23), pages 1-3, XP055825959, relates to the estimation of virus concentrations based on the monitoring of sewage.

Thomas Macaulay: "Startup will map the coronavirus spread by looking at poo", , 20 March 2020 (2020-03-20), pages 1-4, XP055825962, relates to the mapping of a pandemic by analyzing data from sewage samples.

### DISCLOSURE OF INVENTION

According to aspects of the present disclosure, a point of care device, comprises a chamber configured to contain a swab, a buffer delivery system that introduces a buffer into the chamber, a source to lyse cells collected onto the swab placed in the chamber, and a probe delivery system that introduces a probe into the chamber. In some embodiments, the point of care device can directly output a test result, e.g., via output to a portable electronic device such as a smartphone or via other approach.

According to further embodiments, a process for using waste water to determine infection of a region is disclosed. The process comprises receiving a sample of waste water. The sample is associated with location information of where the sample was taken. Diagnostics are then performed on the sample to determine viral load information associated with the sample. Further, a risk assessment is determined based on the viral load information and the location information. The viral load information and the risk assessment is displayed in conjunction with a map of the region.

According to yet further embodiments, a process for generating a database to identify pathogens is provided. The process comprises generating a database that includes reference sequences of pathogens found in the human biome, non-pathogens found in the human microbiome, and subsets of genes known to be virulence factors, antibiotic resistance genes, genes encoding unique structural features, or host response factors. Identified regions within the sequences are used to identify a pathogen in broad characteristics, and an incomplete sequence associated with a pathogen not associated a complete reference sequence in the database is added to the database. Further, the incomplete sequence is based on the broad characteristics.

### BRIEF DESCRIPTION OF DRAWINGS

FIG. 1 is a schematic illustration of an assay cartridge, process workflow, cartridge reading, and other capabilities of a digital droplet point of care device, according to various aspects of the present disclosure;
FIG. 2 is an example of a swab device, according to various aspects of the present disclosure;
FIG. 3 illustrates an example extraction and droplet making module for use with the assay cartridge of FIG. 1, according to various aspects of the present disclosure;
FIG. 4 is an example illustration of a digital PCR output, according to various aspects of the present disclosure;
FIG. 5 illustrates an example diagnostics and therapy pipeline;
FIG. 6 is a diagram illustrating a database to identify pathogens, according to various aspects of the present disclosure;
FIG. 7 is a diagram illustrating a process for comparing virus risk models, according to various aspects of the present disclosure;
FIG. 8 is a flow chart illustrating a process for using wastewater to determine infection of a region, according to aspects of the present disclosure;
FIG. 9 is an example dashboard for a bioanalysis tool via wastewater analysis, according to various aspects of the present disclosure;
FIG. 10 is a map panel of the example dashboard of FIG. 9, according to various aspects of the present disclosure;
FIG. 11 is an overlay panel of the example dashboard of FIG. 9, according to various aspects of the present disclosure;
FIG. 12 is a census tab of an analysis panel of the example dashboard of FIG. 9, according to various aspects of the present disclosure;
FIG. 13 is a site tab of an analysis panel of the example dashboard of FIG. 9, according to various aspects of the present disclosure;
FIG. 14 is a component of the site tab of the analysis panel of the example dashboard of FIG. 9, according to various aspects of the present disclosure;
FIG. 15 is a heatmap of the site tab of the analysis panel of the example dashboard of FIG. 9, according to various aspects of the present disclosure;
FIG. 16 is a sample results tab of the analysis panel of the example dashboard of FIG. 9, according to various aspects of the present disclosure;
FIG. 17 is a dual Krona chart of the sample results tab of the analysis panel of the example dashboard of FIG. 9, according to various aspects of the present disclosure; and
FIG. 18 is a table of organisms in the sample results tab of the analysis panel of the example dashboard of FIG. 9, according to various aspects of the present disclosure.

### MODES FOR CARRYING OUT THE INVENTION

Initially, it is noted that while COVID-19 and SARS-CoV-2 are discussed as specific examples herein with regard to point of care testing, other viruses and/or pathogens may be detected using the methods described herein. Other pathogens may require corresponding adaptations to components described herein.

Conventional diagnostic testing for many viruses puts healthcare workers and other citizens at risk. Furthermore, most diagnostic assays use the same reagents, which could lead to a shortage in the market, e.g., in the case of a large scale event.

However, embodiments herein provide a point of care solution for testing for pathogens that can include viruses. This allows, for example, an at home solution for diagnostic testing that can be delivered to the home of an individual in need of testing. This reduces the potential of transmission to healthcare workers and the general public. The sick individual is no longer required to leave their home, drive to a test site, wait in a doctor's office, and have a healthcare worker sample them for the test. Also, home testing will reduce the amount of personal protective gear and burden on the health care system that is being overwhelmed by people who want to and/or need to be tested.

Embodiments herein produce rapid diagnostic testing, without relying upon an expensive and complex analysis instrument.

Embodiments herein provide a device that includes all steps necessary to provide point of care specimen collection, analysis, and result reporting, in a single device to enable at home diagnostics to be done by anyone. Rapid at home testing is important not just during the exponential growth phase of a pandemic, but after a pandemic is under control, at home diagnostics is desirable to avoid a second burst of infections. This device will enable anyone who feels sick to test themselves and then take appropriate action, e.g., self-isolate, to avoid recurring epidemics.

By enabling home testing, people who have a condition (e.g., COVID-19) presenting with mild symptoms can test themselves. For instance, due to reagent and PPE shortages, an individual may have to exhibit clear symptoms (e.g., an individual may have to have a fever, dry cough, and trouble breathing) to be tested. This can leave people circulating in the population who could unwittingly spread disease. Furthermore, home testing will reduce the burden on our healthcare system and supply chain of PPE and reagents.

According to an embodiment of the present invention, molecular methods are provided that can be automated in a portable test device, e.g., a single use disposable handheld test device. Someone who thinks they have a condition such as an infection (e.g., SARS-CoV-2) can use a swab to collect a sample specimen (e.g., swab the back of their throat such as on the tonsils, collect a nasal swab, saliva swab, etc.), and place the swab in a chamber of the test device. After inputting the swab, an assay begins. In some embodiments, the chamber is a locking chamber. For instance, in an example implementation, a microfluidic device locks the swab in place. By locking the swab in place, the test is sealed. Regardless, the test device introduces a buffer into the chamber (e.g., floods the chamber with a buffer), and applies heat (and/or a chemical approach) to lyse the cells collected onto the swab. Moreover, probes are introduced into the chamber, and an output of the test result is provided. In some implementations of the present embodiment, the whole process, from sample specimen collection to test result answer, can take 30 minutes or less.

According to another embodiment herein, a workflow includes specimen collection, heat treatment (e.g., heat lysis), probe detection, and amplification to detect a targeted pathogen or class of pathogen (e.g., SARS-CoV-2) in a single reaction chamber.

### Point of Care Device

In an example embodiment, a testing device is implemented as a Point of Care device (POC). In general, the device includes two subsystems, including a cartridge and a droplet module, each is discussed below.

### Cartridge

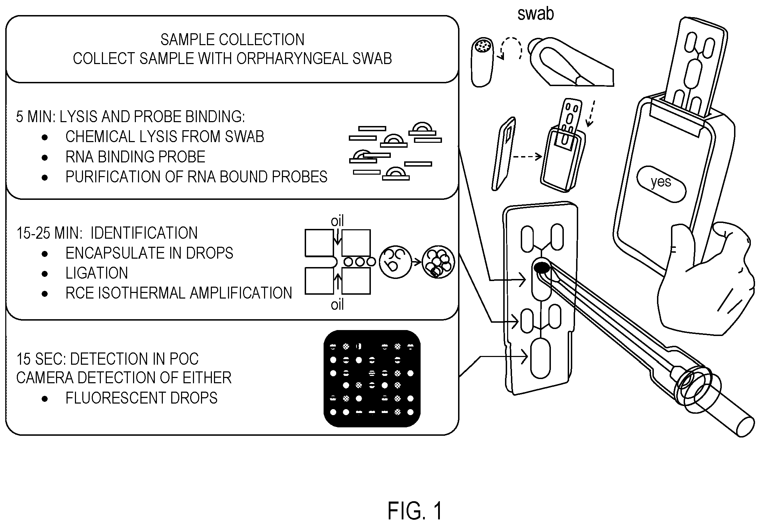

Referring now to the drawings, and particular to FIG. 1, a POC utilizes an assay cartridge as illustrated. The assay cartridge is provided for detecting one or more targeted pathogens, e.g., (a virus such as SARS-CoV-2).

The cartridge has an extraction chamber in which a swab is positioned. In an example embodiment the swab locks in place. Once a swab is placed in the chamber, a sample is released and lysed from the swab and is then mixed with reagents.

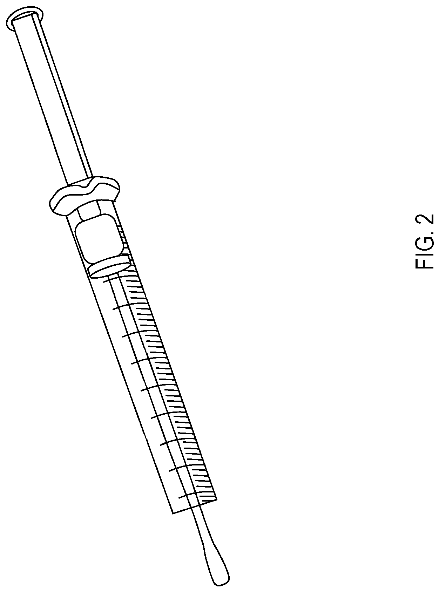

As described more fully herein, the POC device includes provisions for sample collection (e.g., collecting a sample with an oropharyngeal swab). By way of example, the POC can comprise a chamber configured to contain a collected swab. In this regard, the POC can use any chamber configuration as set out more fully herein. Moreover, depending upon the implementation, the swab can contain a specimen collected by a variety of means, e.g., throat swab, nasal swab, saliva swab, etc. Referring briefly to FIG. 2, an example swab device is illustrated. The device can utilize a luer lock or fitting to meet the chamber of FIG. 1.

The POC device also includes provisions for lysis and probe binding.

In an example embodiment, the POC can include a buffer delivery system that introduces a buffer into the chamber, a probe delivery system that introduces probes into the chamber, a heat source that applies sufficient heat to lyse cells collected onto the swab placed in the chamber, combinations thereof, etc. Moreover, as used throughout, the device can include one delivery system that delivers all components (e.g., buffer, probe, etc.) each component can have its own delivery system, a delivery system can share between two or more components, combinations thereof, etc.

The POC device can also support identification, e.g., via encapsulation of drops in ligation, RCA isothermal amplification, color changing dye, combinations thereof, etc. In an example embodiment, the identification process can take between 15-25 minutes.

The POC can also support detection. For instance, a camera, e.g., from a smartphone, can be used to detect indicators such as fluorescent drops Moreover, in some embodiments, e.g., where a color-changing dye is used, no camera/smartphone may be required. In an example embodiment, detection can be carried out in approximately 15 seconds.

In an example implementation, the assay targets a virus (e.g., a SARS-CoV-2 nucleocapsid gene) to detect viral load as well as the human RNaseP gene for an assay control. The nucleocapsid gene can be the target of the diagnostic assay. In an example embodiment, the entire assay takes place on a disposable microfluidic chip and the result is read by a camera of a common smartphone.

This device and readout scheme reduces diagnostic device cost and reliance on sophisticated hardware components, which may suffer supply chain disruptions in periods of crises. Also, the POC device provides the general population access to a diagnostic with an easy to comprehend result, e.g., an easy "yes/no" result in under 30 minutes.

In an example embodiment, a microfluidic subsystem is implemented as a cartridge that facilitates a lock in place swab, a single chamber that is flooded with buffer and heated to release RNA, with automated timed release of probe reagents, removal of buffer, and influx of isothermal amplification and detection reagents.

### Droplet Module

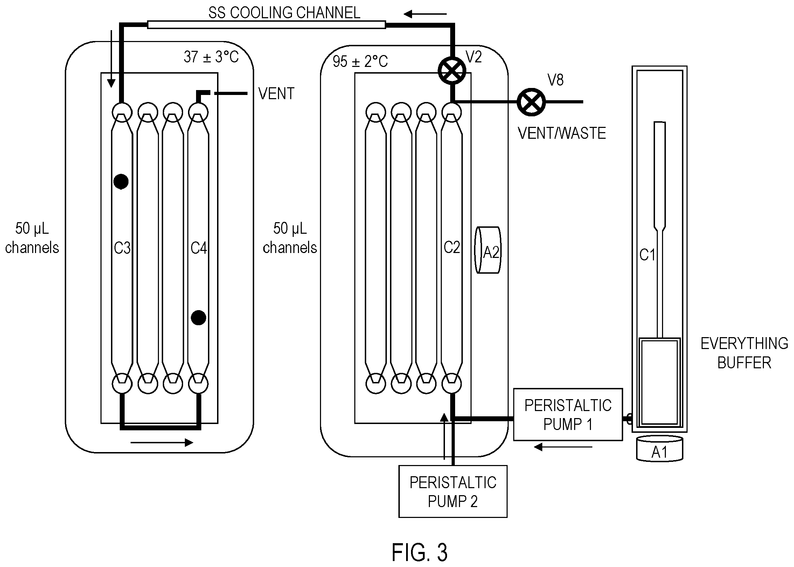

Referring to FIG. 3, in an example embodiment, a droplet module can be utilized. The droplet module comprises a small durable processor (e.g., microcontroller) and an extraction and dropmaking (ED) module. The module may also include any other necessary hardware, e.g., microfluidic chip(s), heating elements, pumps, valves, tubing, hydrophobic filters, buffer and lyophilized reagent storage reservoirs, etc.

According to aspects of the present disclosure herein, a microfluidic chip can be made of Polymethylmethacrylate, TOPAS material, etc. Valves and hydrophobic filters can be implemented to reduce a need for precise fluid volume metering, to increase device reliability, etc.

In some embodiments, digital droplet technology complements assay chemistry to deliver superior detection performance in a fraction of the time of qPCR. The assay cartridge FIG. 1 inserts into a module, which is illustrated in FIG. 3.

Referring generally to FIG. 1 through FIG. 3, in an example embodiment, a dropmaker enables an aqueous stream to contain a target sequence and all necessary reagents for digital PCR.

The sample is passed through the droplet maker to compartmentalize the template and reagents into individual, "digital" droplets. This compartmentalization increases the effective concentration of the target oligos, e.g., by a factor of 10⁶, dramatically increasing sensitivity. This also reduces the potential for competing targets, thereby improving specificity. In some embodiments, a detection signal is concentrated in "positive" droplets, significantly improving the signal-to-noise ratio, further increasing sensitivity.

In some embodiments, the droplets are collected and stored in a reservoir on the chip. The cartridge is then moved to the amplification module, e.g., a simple constant temperature heater, raising the temperature of the collected specimen to perform amplification. After amplification, the cartridge is placed in a mount attached to a camera, e.g., a common smartphone, which is used to collect one or more images.

### Chamber

In other embodiments, digital droplet technology is not utilized. Rather, a chamber is utilized to facilitate a bulk reaction.

### Example Considerations

According to yet further aspects herein, an embodiment can utilize two (or more) probes physically bound to a structure (e.g., a glass slide, a flow cell, etc.). In this regard, when a pathogen nucleic acid is present and the ligase is present, detection is carried out by ligation, e.g., in a microarray-type configuration. A normal polymerase can be used to amplify the spot on the slide, e.g., in a manner analogous to that carried out in an Illumina flow cell.

As noted more fully herein, any disclosed techniques herein can be used in combination with other techniques, structure, methods, etc., in any combination. For instance, a two-probe technique can be utilized with a digital droplet technology, a bulk chamber, etc.

### Example Output 1

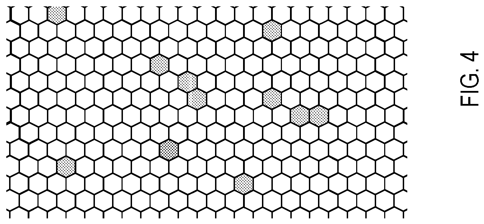

Referring to FIG. 4, an example digital PCR output is illustrated. In an example embodiment, a companion app provided on the smartphone analyzes the image(s), e.g., by counting the bright droplets in the image to determine the concentration of the template of interest, with no calibration necessary. This workflow enables high throughput with simple equipment. In example embodiments, a single set of modules can be capable of handling 10-12 samples per hour by simply adding more ED modules, the system could process up to 50 POC tests an hour.

In some embodiments, processes are automated so that an end-to-end analysis and reporting system is provided.

A sample enters the device, e.g., as a nasal swab or saliva. The device automatically extracts the nucleic acid by heat and/or chemical lysis. Lysis can be carried out for instance, on a chip or other structure as described more fully herein. Then, a probe (padlock probe, two or more probes bound to a slide, flow cell, etc.), binds. The bound probe is ligated and then amplified, such as by using an isothermal polymerase (e.g., Bst or phi29). In an example embodiment, a linear product generation causes a pH shift in the liquid that is then detectable by human eye.

### Fluorescent Output Example

An alternative method uses a two part probe which circularizes. One part is a padlock probe having a gap that is filled in by the second part of the probe. This second part is biotin labeled. The two part probe hybridizes to the pathogen RNA and is then ligated by Chlorella ligase. Streptavidin beads can be used to move the ligation product to a Rolling Circle Amplification chamber. Rolling circle amplification occurs and a secondary probe binds the RCA product as it is generated making it dsDNA. A contrast, such as SYBR GREEN I, is utilized to visualize the product as green fluorescent light under UV.

### Miscellaneous

Referring to FIG. 1 through FIG. 4 generally, the POC device is simple to use, rapid, and scalable. Thus, the POC can be used for rapid screening at home, at entryways to public buildings (e.g. businesses, airports, etc.). Moreover, the POC enables real-time surveillance of a virus (e.g., COVID-19).

Molecular detection of a virus (e.g., COVID-19 and other viral pathogens) is currently performed by real-time Polymerase chain reaction (qPCR). However, qPCR limits the throughput of the detection as it requires RNA to be extracted and purified from the samples, taking time and reagents prior to conducting the diagnostic assay. qPCR also requires specialized hardware for precise and repeated thermocycling while simultaneously monitoring the concentration of template in real time. This limits the throughput, as the detection and the amplification are linked. Further, the need to thermocycle bottlenecks amplification as there is time lost for every temperature change.

In contrast, embodiments herein provide a POC that uses an isothermal amplification method. This drastically reduces processing time by removing the need to thermocycle. In addition, the use of digital droplet technology provides rapid turnaround and sensitivity, enabling detection from a nasal swab or saliva instead of an invasive nasopharyngeal swab.

In some embodiments, the reaction is encompassed in a disposable cartridge that inserts into a handheld, simple chassis that can be stored at home for further use. The readout is simple and obvious so that the user will know definitively if they have the infectious disease or not. An example application is to test for COVID-19.

In use, a user can put a swab in the cartridge, hit start, and then visibly see a color change, fluorescence, or readout on their smartphone display that indicates positive or negative results. By having an affordable home diagnostic test system, one can reduce the spread of infectious diseases by identifying everyone who is sick, not just those who have been admitted to the hospital or have a fever.

In an example embodiment, total test time is approximately 30 minutes or less. In this regard, the POC can extract viral RNA in under 5 minutes. In this regard, a device attachment houses a swab and locks into a sample input port to begin automated washing with lysis buffer, as shown in FIG. 1. Some embodiments use heat lysis, e.g., at 95°C. Other embodiments use chemical lysis. To control RNA concentration, commercially available Kapa RNA Beads can be utilized. These beads reversibly bind RNA, allowing an ability to meter the template before droplet formation to avoid saturating the assay.

In an example configuration, a cartridge can accommodate an initial lysis buffer volume of 200 µL with concentration of RNA down to approximately 15 µL prior to detection reactions. In some embodiments, the cartridge has no more than four inputs.

In some embodiments, a device is comprised of subsystems, including a microfluidic cartridge where buffer and lyophilized reagent storage reservoirs house components. In example embodiments, fluid communication can be driven by a notional pump, and a heating architecture can be integrated, e.g., where chemical processing is not implemented.

A dropmaker module, where utilized, can co-encapsulate single template molecules bound to padlock probes with necessary reagents that can be modified to optimize detection of specific test conditions (e.g., a virus such as SARS-CoV-2). In an example configuration, if a reservoir can hold 10⁶ droplets, the corresponding dropmaker module should produce and store 10⁶ droplets in a predetermined amount of time, e.g., under 5 minutes, and preferably 1 minute or less.

In an example configuration, a one-chamber probe hybridization, ligation, and amplification approach is implemented.

Further aspects of the present disclosure provide a rapid, and easy to use method for sample collection and diagnostics, which can interface with biosurveillance networks and physicians, e.g., via online connections, to best determine if someone should self-isolate, seek medical care, or take other action. Examples of biosurvelliance are described in a non-limiting manner herein, e.g., in the context of wastewater treatment plant screening. Moreover, because a smartphone is utilized in some embodiments, cellular, wifi and related technologies can link to cloud-based data source to compile accurate, near-real time counts of uses, diagnostic results, etc. As such, trends can be tracked and predicted, accurate infection counts can be made, etc. Moreover, while doing so, homebased diagnostics protect our frontline healthcare workers, as they do not have to be exposed during sample collection.

### Epigenetic Diagnostics and Therapeutics for Environmental Exposures to Harmful Substances

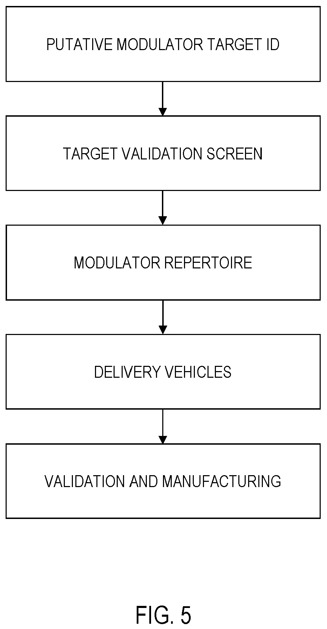

Referring to FIG. 5, there are some instances where it may be desirable or practical to focus on host factors instead of the pathogens.

For instance, pathogens and other exposures effect the epigenome (or other putative modulator target identification) even before symptoms are observable. By determining targets in the epigenome that are affected by different pathogens or exposures, diagnostics and therapeutics can be designed to detect and counter these threats. Accordingly, aspects of the present disclosure screen for epigenetic regulators that are effected by exposures to enable targeted treatments.

More particularly, fusions of epigenetic regulators to dCas9 or zinc finger proteins (found in a modulator repertoire) enable targeted removal of methylation or histone modifications that are the molecular basis of certain diseases (which can include addiction or neurologically based diseases). These fusion proteins, or epieffectors, are then encapsulated or bound to delivery systems (i.e., delivery vehicles) made up of either magnetic nanoparticles, liposomes, or functionalized microbubbles that can be transfected into specific cell types for directed and specific modification of targets.

This therapy treats neurological diseases without broad spectrum effects across the human body, reducing side effects. Several diseases or disorders are caused by epigenetic changes in response to exposures in the environment. By removing these epigenetic modifications in a targeted manner, the disorder can be removed without debilitating side effects. Furthermore, for disorders such as depression or anxiety, the patient would not need to be on the medication long term, as once all cells are reverted to the normal methylation pattern, the disorder will be cured.

Notably, the epieffector is targeted to specific locations in the genome due to guideRNAs or specific zinc finger motifs. Moreover, aspects herein provide an effective delivery mechanism that enables cell specificity for localizing the epieffector molecules.

In response to stress, the promoters of several genes involved in the hypothalamic-pituitary-adrenal (HPA) axis become hypomethylated causing a shift in gene expression. As such, aspects herein provide a high throughput screening tool for methylation and gene expression changes at these promoters by small molecules, epieffectors, or exposure to microbiome by replacing the genes with the luciferase gene in relevant cell lines. The modified cells will grow in culture and when exposed to small molecules, epieffectors, microbiome, probiotics, or other therapeutics that modulate the expression of these genes or methylation of the promoters, a quantifiable change in emitted light will occur. Since this light emission can be read in 96 well or 386 well plate format, such emission will enable screening of small molecule, probiotic, epieffector, therapeutic, or microbiome libraries to discover new candidates for treating depression, anxiety, post-traumatic stress, and other stress related disorders. These candidates may then be validated and manufactured if desired.

Notably, aspects of the present disclosure include luciferase reporter fusion to promoters known to control the hypothalamic-pituitary-adrenal axis. Moreover, aspects herein provide microbiome or other library for screening, and leverage the use of a spectrophotometer or other device for reading results.

### Genetic Target Identification System for Infectious Disease Diagnostics Development

Molecular diagnostics only detect known pathogens. However, according to aspects of the present disclosure, novel pathogens (i.e., non-known pathogens) may be detected by targeting conserved regions of a genomic sequence that can infer function. Thus, a need for designing new diagnostic tests when facing a novel epidemic or outbreak is reduced, which improves response time in the field and more accurate treatment of infected patients.

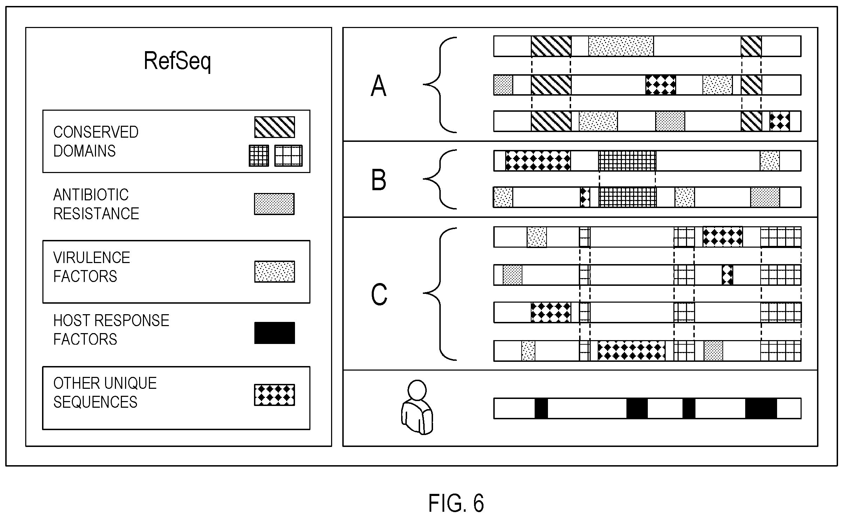

To help determine which sequences will provide information necessary for a medical diagnosis of an infectious disease, a database includes genomic reference sequences of pathogens found in the human microbiome and non-pathogens found in the human microbiome. Moreover, the database includes subsets of genes known to be virulence factors, antibiotic resistance genes, and host response factors. Further, machine learning is used to identify conserved domains within groups of pathogens (through interpretation of conserved domain function) to allow for identification of novel pathogens, which may not be in the database. A sample of such a database is shown in FIG. 6.

Thus, a novel pathogen may be detected from a diagnostic test using conserved domains. In other words, regions of the genetic sequences that indicate a function may be identified via machine learning. Those regions may then be used to identify a pathogen in broad characteristics based on virulence factors, genes encoding unique structural features, and the host response to the infection.

Therefore, based on the database of genetic information, machine learning will identify and categorize genomic sequences to identify the best regions to be used as targets of molecular assays that determine a diagnosis of a patient. This will enable a molecular diagnostic to identify novel pathogens as well as known pathogens, and reduce the need for new diagnostic tests to be developed during novel outbreaks.

### Wastewater Sampling

According to embodiments of the present disclosure, wastewater treatment plants are utilized as a viable source for biosurveillance. More specifically, wastewater influent is analyzed to determine locations of outbreaks. In certain locals such as large cities, multiple wastewater treatment plants are utilized. In this regard, an outbreak location can be determined down to neighborhoods that flow to one wastewater plant. In an illustrative example embodiment, metagenomic sequencing and/or targeted sequencing can be used to determine what pathogens are present in different cities. To maximize the information gathered from wastewater-based testing, targeted whole genome sequencing should be used in tandem with real-time Polymerase chain reaction (qPCR) (an in some cases, untargeted RNA sequencing) to determine the viral load and identify the dominant viral strain in circulation, including mutations that could affect diagnostic or therapeutic strategies. Furthermore, wastewater-based epidemiology (WBE) implemented within catchments at the neighborhood or building level will enable a more refined map of COVID-19 hotspots.

Outbreak tracking during epidemics and pandemics is important to identify communities at risk for disease and to prevent further spread. According to aspects of the present disclosure, tracking pathogens that may lead to outbreaks in cities includes periodic sampling of wastewater from wastewater treatment plants (WWTP) to identify when an outbreak has reached a city and how prevalent the pathogen is in communities of the city. Governments and public health officials who need to make policy decisions can use the information gained from tracking the pathogen to develop policies such as mandating social distancing measures or allocating diagnostic tests and therapeutics where they are needed most. One disease that could be tracked from human waste is the coronavirus disease 2019 (COVID-19). While COVID-19 and SARS-CoV-2 are discussed as a specific example herein with regard to waste water treatment plant sampling, other viruses and/or pathogens may be detected using the methods described herein.

Viruses (e.g., SARS-CoV-2 (the causative agent of COVID-19)) spread worldwide and new measures are needed to track the presence of the viruses, identify any mutations that occur during spread, and monitor containment efforts. A recent medRxiv publication (https://doi.org/10.1101/2020.03.05.20030502), has indicated that high RNA concentrations of SARS-CoV-2 have been detected in stool samples from 49% of patients at levels and time durations comparable to those found in sputum samples from hospitalized patients. This suggests that wastewater, which contains stool, could be used as a surrogate to sputum samples to monitor SARS-CoV-2 presence in populations.

Samples associated with a city's sewer system are taken from incoming, untreated sewage and include data associated with a location from where the sample was collected and/or sequenced in real-time (e.g., using MinION) to determine an abundance of a virus (e.g., SARS-CoV-2) in the samples (i.e., a viral load of the samples). Further, the sequencing may also be able to detect mutations in the virus.

Using a geographic information system (GIS), the samples are overlaid on a map of the associated city's sewer systems (including where those systems connect to each city's wastewater treatment facility) and Census geographies (including population demographic attributes) to identify potential areas at high risk for an outbreak. A software application that integrates the sampling results and map layers is used to monitor levels of SARS-CoV-2 in the city (and communities associated with the city) to identify whether the viral load is increasing or decreasing and how the viral load might impact risk to that city, community, or both.

Thus, the software application allows users to monitor spread of virus infections (e.g., COVID-19) throughout the city, locate regions/communities at highest risk for spread of infection, and identify gaps in testing where an estimated infection rate is different than expected.

COVID-19 tracking to date is relying on samples from confirmed cases, but a lack of testing means that many of the cases are undetected and spreading throughout the community. However, using the methods described herein, samples of waste water can be used detect infection and spread.

Waste water itself is a combination of human waste, water, tissue paper, food waste, and other items flushed down drains. Further, there is a lag between the time stool is excreted to when the stool reaches a waste water treatment plant. Thus, the viral load may fluctuate based on an amount of time between excretion and sampling/diagnosis, which should be taken into account when detecting a viral load within the sample. During that time, the stool is diluted and mixed with the other items listed above. Thus, when detecting a viral load within a sample, the dilution of the sample must be factored into any calculation.

Sample locations are determined based on the associated city/community/region (e.g., waste water treatment plant, buildings throughout a community, hospitals, nursing homes, etc.). Moreover, location information is associated with the samples. For example, the location information may be latitude/longitude coordinates, street references, etc. Other information such as metadata (e.g., time collected, offset from location data, etc.) may also be associated with the samples. Moreover sample locations are not required to be fixed. For example, a sample location may be updated and/or moved based on an unforeseen difficulty (e.g., flood, conflict, etc.).

When determining sample locations for a region (i.e., sampling plan), several factors may be considered (e.g., spacing of the sampling grid (square, triangular, or rectangular), size of a detectable hot spot, and a hit probability). With two of these factors held constant, software may be used to solve for the third factor. Further, the software may use a Mann-Kendall test to determine if viral load is monotonically (not necessarily linearly) increasing or decreasing over time. Moreover, boundaries of contamination can be established and used to determine if SARS-CoV-2 exists beyond a known boundary (such as where it can reach consumers). This approach uses multiple increment (MI) sampling. The boundaries resulting MI sampling can be fully or partially enclosed.

After a sample is taken, the sample is processed to determine viral load, and a software application conveys the results on a map of the region on a display. In some embodiments, the software application allows for several layers of information to be shown, where each layer may be toggled on or off independently of other layers. For example, layers may include a current viral load, viral load over time, a street map, city/state borders, sewer systems, census information, sampling locations, etc. The software application may also display summary information as a mouse hovers over different parts of the display.

In some embodiments, the software application includes a risk attribution model that uses viral loads, sampling locations, and the demographics of the population to pinpoint the areas of high risk for COVID-19 and how those areas are changing over time. Furthermore, by reconciling SARS-CoV-2 concentrations at sampling sites with locations of confirmed COVID-19 cases, it should be possible to identify gaps in testing (i.e., regions that are showing higher than expected infection rates). The risk at sampling locations is estimated using a logistic regression model and then smoothed over time and space (e.g., using a gaussian process model, using a spline interpolator, etc.). Thus, users can identify any concerning trends and act accordingly.

An empirical risk attribution model includes rates of upstream COVID-19 cases as a function of viral loads at sampling locations and demographics of the upstream population. This model provides a geospatial map of COVID-19 risk over time, as viral loads change.

By assuming most of the SARS-CoV-2 RNA in sewage results from fecal matter and average amount of virus shed per individual, a rough estimate of the number of infected individuals upstream of each sampling location can be created.

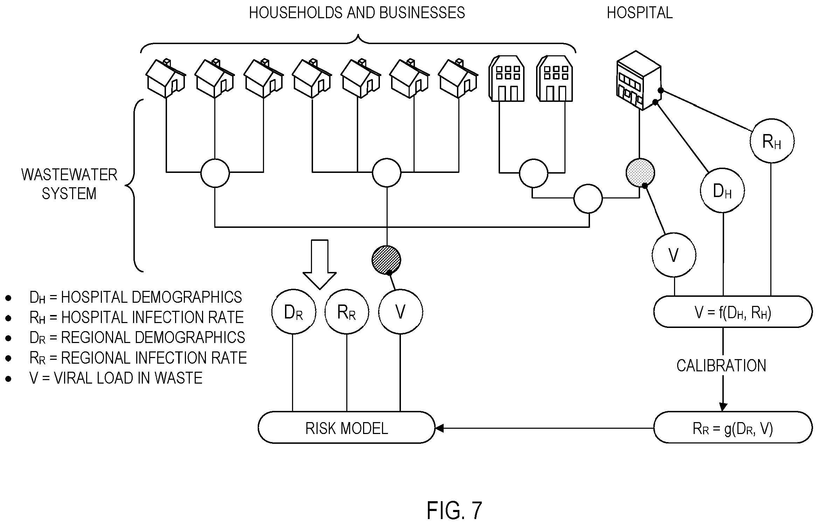

If a single or small set of sampling locations have a well-characterized upstream number of exposed individuals, e.g., a sampling location servicing primarily a hospital, these data can be used to produce an empirical estimate of the relationship between SARS-CoV-2 RNA quantity and number of infected individuals, which can be used to calibrate the first principles COVID-19 model. Estimates of number of COVID-19 infected individuals from this first principle model are compared to the empirical wastewater-based COVID-19 risk model previously described (see FIG. 7).

For example, in FIG. 7, a hospital has accurate information about demographics (D_{H}) and infection rate (R_{H}). Viral load (V) is a function of the demographics and infection rate, and by sampling at the hospital level, a hospital-level model can be used to predict the viral load given the demographics and infection rate. The hospital-level model is then inverted to predict infection rate (R_{R}) given the viral load and population demographics (D_{R}) of the region. Comparing the predicted infection rate to the measured (actual) infection rate will indicate whether the region has been undertested or may be experiencing an increase in infections.

By combining the wastewater-based COVID-19 risk model and the first principles COVID-19 model with epidemiological models emerging that predict hospitalizations and ICU and ventilator usage for individuals with COVID-19 expected hospitalization and ICU and ventilator usage can be further estimated. These estimates can be compared with empirical data for the local community and with other COVID-19 prediction models to assess the wastewater-based COVID risk model.

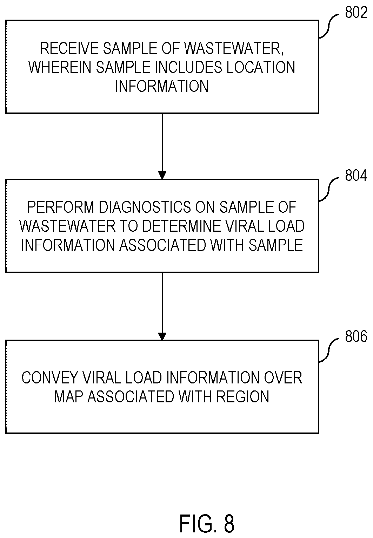

FIG. 8 is a flow chart illustrating a process 800 for using wastewater for determining a viral infection of a region. At 802, a sample of wastewater is received. For example, the wastewater may be received from any location around a geographical region, as discussed above. The wastewater sample also includes location information that indicates where the sample is from. The location information may be in any form (e.g., electronical, written, etc.) and may include information associated with GPS coordinates, latitude/longitude, reference to a specific location (e.g., Hospital A, Sewer Access 1, etc.), etc., or combinations thereof.

At 804, diagnostics are performed on the wastewater sample to determine a viral load of the sample to obtain viral load information. As discussed above, the diagnostics can be performed in a two-part process including performing real-time polymerase chain reaction on the sample and then independently performing targeted sequencing based on a genome of a virus associated with the viral load. In some embodiments both diagnostics are performed on every sample. In various embodiments, the real-time polymerase chain reaction diagnostics can be performed as a gate keeper to subsequently pass the sample onto the targeted sequencing. In numerous embodiments, the targeted sequencing can be performed as a gate keeper to subsequently pass the sample onto the polymerase chain reaction diagnostics. As mentioned above, the diagnostics can account for dilution of the sample, an amount of time that has passed between collecting the sample and diagnosing the sample, or both.

At 806, the viral load information is then conveyed on a map associated with the region. For example, the map may have several layers, and one of the layers may be viral load information. A software application can display the map and the associated layers on a screen, as discussed above. More information about each specimen location may be displayed when a user selects the specimen location on the map, hovers over the specimen location on the map, etc. A geographical layer of the map may include satellite photographs, a representation of the location, or both.

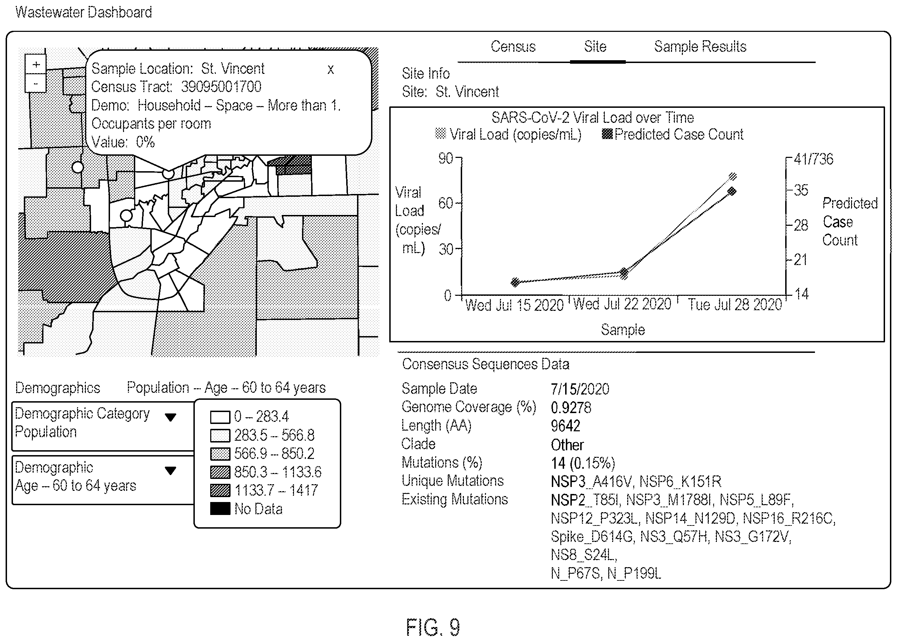

The map can also be used as part of a dashboard tool (FIG. 9) that allows a user to navigate through regions graphically. The dashboard can be a bio-surveillance tool that is used to track an incidence and spread of pathogens that can be quantified through wastewater analysis, as described herein. The tool accommodates both qPCR and whole-genome total RNA sequencing of wastewater samples collected at sites anywhere in the region of interest. Through this tool, a user can view information at both a site (specimen location) level and a region level. Site-specific information includes metadata about the wastewater samples, summaries of sequencing results, and the ability to view those results at different time points when repeated samples are taken from the same location. At the regional level, the WPTD also provides an overlay of demographic variables of interest at a census tract resolution, allowing the user to understand the pathogen detections and prevalence in the context of the populations being affected.

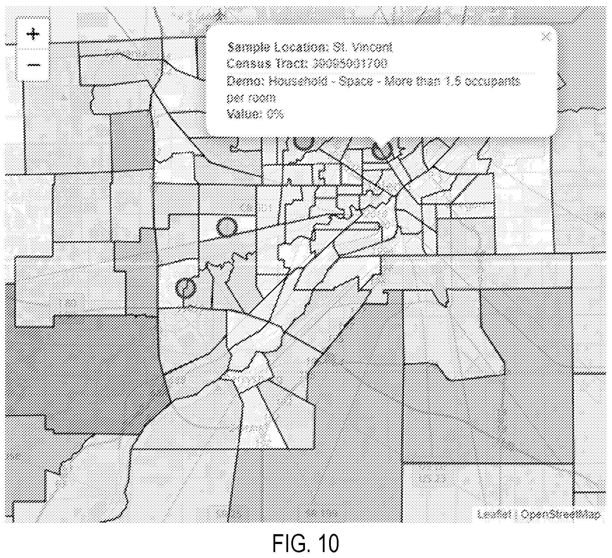

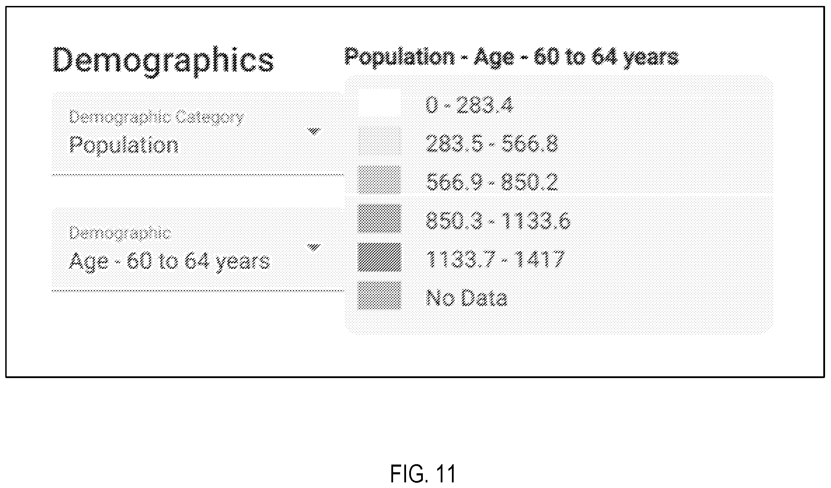

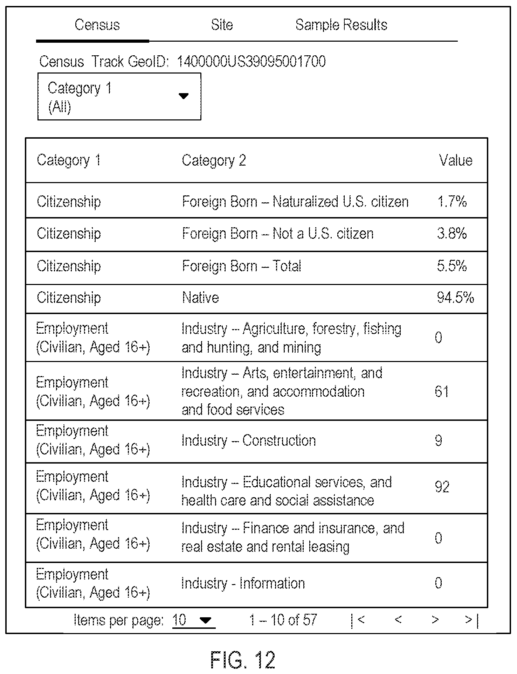

Further, the dashboard can be divided into several panels. For example a first panel may be a map panel (FIG. 10) that shows a map of the region of interest and the distribution of wastewater sampling locations (sites) available for exploration. The region can be divided into subregions (e.g., census tracts) and can also display an overlay of a demographic variable (e.g., population by age, occupation, etc.) selected from an overlay panel (FIG. 11). Each of the sampled sites may be denoted (e.g., appear as a red circle) and may be selected by the user to populate an analysis panel (FIGS. 12-18) with information and analysis results for that site. A pop-up can also appear on the map panel to show the user which site (i.e., sample location) that is selected and share summary-level metadata about the site.

In the overlay panel (FIG. 11), the user can select between multiple pre-populated lists of population demographic variables that may be associated with or affected by the spread of pathogens in the community. These variables are captured at the subregion level (e.g., census tract level) and used to produce the overlay in the map panel. As shown in FIG. 11, a demographic category pulldown menu is set to POPULATION and a demographic pull down is set to AGE: 60 TO 64 YEARS. To the right, a color coding scheme illustrates population density of the desired demographic category. The color-coding scheme is then applied to the map panel for the user to see. Other demographic categories and demographics may be used as well, each selectable through the appropriate pulldown.

The analysis panel (FIGS. 12-18) is where a majority of the information in the dashboard is displayed and is split into three tabs corresponding to difference scales of analysis being conducted within the region of interest. The first tab may be a census tab and shows a table of population demographics for the census tract to which the selected site belongs. The census tab may also include graphical summaries (e.g., bar charts, histograms, scatterplots, etc.) to further explore the relationship between sets of demographic variables within the census tract. For example, in FIG. 12, a category is chosen through a pulldown, and a table is shown for that category for a selected site. As discussed herein, the site can be chosen through the map panel.

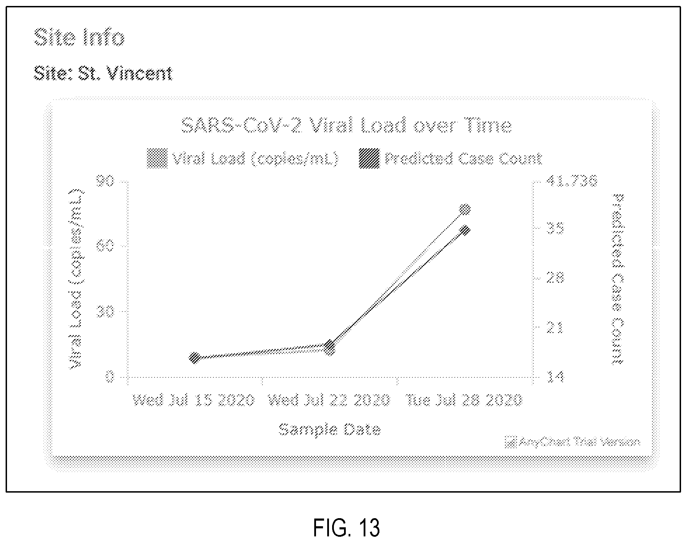

The second tab may be a site tab (FIGS. 13-15) , which contains several site-specific summaries of pathogen detections over time, as measured through qPCR analysis. Along with the viral load, a predicted case count for the site catchment is also provided. For example, this prediction can be made using a statistical Poisson regression model that was trained using wastewater treatment plant data and case counts from a governmental Department of Health . This prediction plot allows the user to understand how prevalent COVID-19 is within the community upstream of the sampling site. Another component in the site tab is a list of consensus sequences observed for each date of the monitoring period at that site. Along with a list of abundances and observed mutations, entries in this list of consensus sequences also indicate connections to known clades of the virus (e.g., Clade GH, which contains the more transmissible UK and South Africa strains). A further component of the site tab is a pair of heatmaps that show abundances of different pathogens identified through whole genome metagenomic analysis of the wastewater samples. The first heatmap shows detection counts for general categories of organisms by date (infection potential over time). By selecting one of these categories, the user can then see a breakdown of detections of specific organisms by date in the second heatmap.

As shown in FIG. 13, the site tab shows site information for St. Vincent hospital viral load over time. A predicted case count is generated from a statistical regression model. However, other sites may be chosen via the map panel.

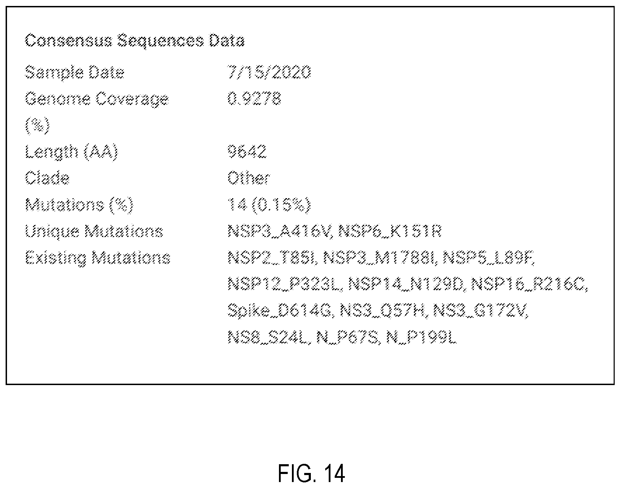

FIG. 14 illustrates a consensus sequences component of the sites tab that includes a list of entries that indicate observed mutations of the SARS-CoV-2 genome, abundances of those mutations, and association with any known clades.

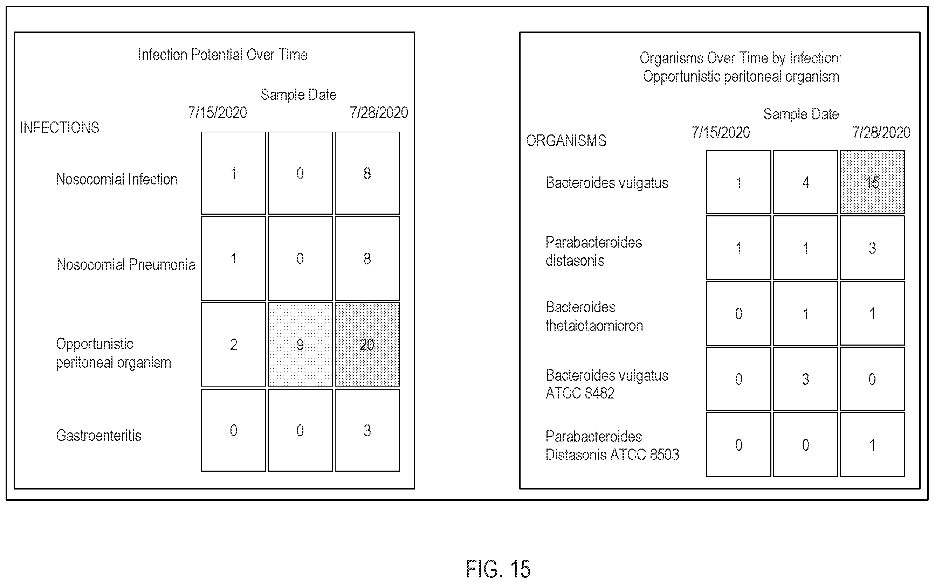

FIG. 15 illustrates heat maps in the site tab to show an abundance of pathogens resulting from metagenomic analysis of the wastewater sample, as discussed herein. All three of FIGS. 13-15 may be shown concurrently in the site tab of the analysis panel or may be divided up however the user wishes for easy access. For example, in FIG. 9, the Site info and consensus sequences data components are shown, but the heatmaps are hidden.

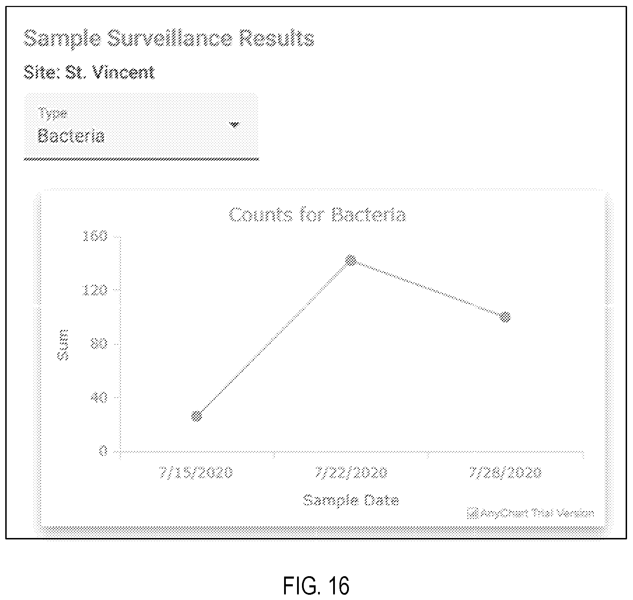

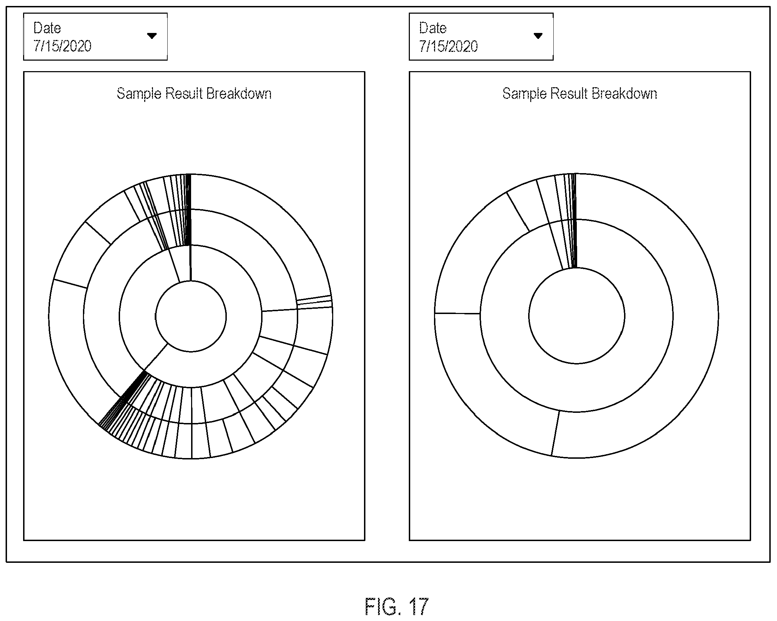

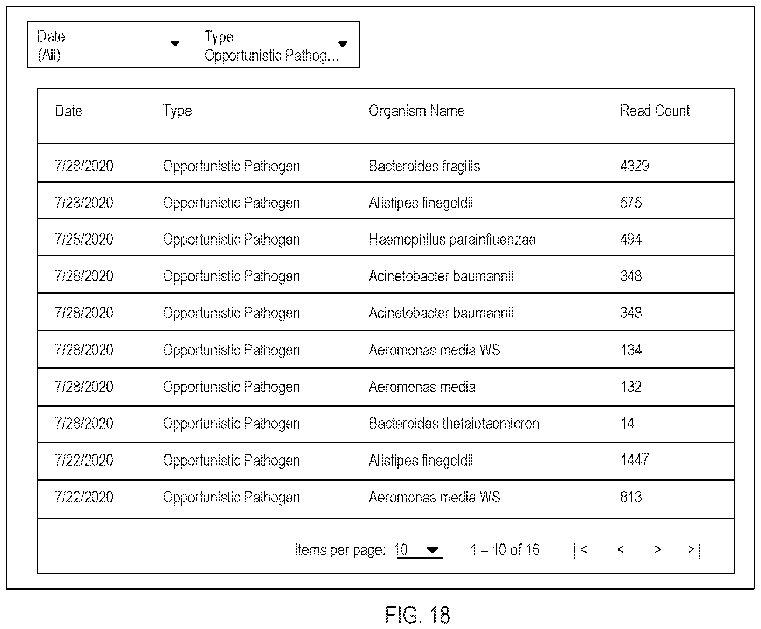

The third tab in the analysis panel is a sample results tab (FIG. 16). A first component within the sample results tab is a plot over time of all contig counts within a category of organisms selected through a pulldown menu. For example, a plot for all bacteria detected in the samples across three sampling dates may be shown. Similar to the SARS-CoV-2 viral load plot in the site tab, the sample results tab gives the user an understanding of how different types of pathogens may be circulating within the community upstream of the sampling site. To better understand the details of the total RNA sequence analysis, the sample results tab also includes an interactive Krona chart (FIG. 17) and associated table of detected organisms (FIG. 18). The Krona chart allows the user to visually explore the abundances of the various organisms detected in the analysis, while the table allows the user to do a similar function through pull down filters.

While total RNA sequencing (RNAseq), targeted whole genome sequencing, and qPCR methodologies can be used to detect SARS-CoV-2 in wastewater, a combination of qPCR to identify viral load and targeted whole genome sequencing to detect mutations provides deeper insights to support public health decisions during pandemic surveillance than qPCR alone. RNAseq, identifies other pathogens present in the samples such as microbes that cause nosocomial infections, periodontitis, gastroenteritis, pneumonia, or cystitis, and can also detect viral pathogens such as SARS-CoV-2, if samples are prepared to capture viral and microbial RNA (e.g., using filtration to filter out large cells (e.g., bacteria) and concentrating RNA in the filtrate (e.g., using centrifugal techniques, polyethylene glycol precipitation, subtractive hybridization, etc., or combinations thereof)) and sequenced deeply, using a microarray, or both to detect pathogens with confidence. The non-targeted surveillance technique would effectively alert hospitals and long-term care facilities to potential issues with nosocomial pathogens or residents that may have infections that need to be treated and can otherwise go undiagnosed such as cystitis due to asymptomatic or recurrent infections. Use in community settings would alert neighborhoods to potential outbreaks (norovirus, Salmonella, COVID-19, etc.) and help public health officials pinpoint restaurants or grocery stores selling contaminated products.

The terminology used herein is for the purpose of describing particular embodiments only and is not intended to be limiting of the disclosure. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

The corresponding structures, materials, acts, and equivalents of all means or step plus function elements in the claims below are intended to include any structure, material, or act for performing the function in combination with other claimed elements as specifically claimed. The description of the present disclosure has been presented for purposes of illustration and description, but is not intended to be exhaustive or limited to the disclosure in the form disclosed. Aspects of the disclosure were chosen and described in order to best explain the principles of the disclosure and the practical application, and to enable others of ordinary skill in the art to understand the disclosure for various embodiments with various modifications as are suited to the particular use contemplated.

## Claims

1. A process for using waste water to determine infection of a region, the process comprising:
receiving a sample of waste water, wherein the sample includes location information;
performing diagnostics on the sample of waste water to determine viral load information associated with the sample;
conveying the viral load information over a map associated with the region by:
generating a first layer associated with the viral load information;
generating a second layer associated with census information; and
displaying the first layer and the second layer independently of each other on a layer associated with a map of the region; and
identifying high areas of risk for a potential outbreak based on the viral load of the samples and the census information.

2. The process of claim 1, wherein performing diagnostics on the sample of waste water to determine viral load information associated with the sample further comprises accounting for a dilution of the sample.

3. The process of claim 1, wherein performing diagnostics on the sample of waste water to determine viral load information associated with the sample further comprises accounting for a time that has passed since stool in the sample has been excreted.

4. The process of claim 1, wherein performing diagnostics on the sample of waste water to determine viral load information associated with the sample comprises:
performing real-time Polymerase chain reaction; and
performing targeted sequencing based on a genome of a virus associated with the viral load.

5. The process of claim 1, wherein conveying the viral load information over a map associated with the region comprises:
generating a layer of the viral load information; and
displaying the layer of the viral load information on a layer associated with the map.

6. The process of claim 1 further comprising:
determining a risk based on the viral load information; and
displaying the risk.

7. The process of claim 1 further comprising:
obtaining demographic information about confirmed cases associated with the viral load;
identifying gaps in testing based on a reconciliation between the demographic information and the viral load information; and
communicating the gaps in testing to a user.

8. The process of claim 7, wherein identifying gaps in testing based on a reconciliation between the demographic information and the viral load information further comprises:
determining a location-based model based on sampling information from that location, demographics, and infection rate;
determining a predicted infection rate for the location by inverting the location-based model; and
comparing the predicted infection rate to the demographic information about confirmed cases associated with the viral load.

9. The process of claim 1, wherein performing diagnostics on the sample of waste water to determine viral load information associated with the sample further comprises using real-time polymerase chain reaction diagnostics performed on the sample as a gate keeper to pass the sample onto targeted sequencing diagnostics.

## Patentansprüche

1. Verfahren zur Nutzung von Abwasser zum Feststellen einer Infektion einer Region, wobei das Verfahren Folgendes umfasst:
Empfangen einer Abwasserprobe, wobei die Probe Standortinformationen einschließt;
Durchführen von Diagnostik an der Abwasserprobe, um der Probe zugehörige Viruslastinformationen zu ermitteln;
Übertragen der Viruslastinformationen auf eine der Region zugehörige Karte durch:
Erstellen einer ersten Ebene, die den Viruslastinformationen zugehörig ist;
Erstellen einer zweiten Ebene, die Volkszählungsdaten zugehörig ist; und
Anzeigen der ersten Ebene und der zweiten Ebene unabhängig voneinander auf einer Ebene, die einer Karte der Region zugehörig ist; und
Identifizieren von Gebieten mit hohem Risiko für einen möglichen Ausbruch auf der Grundlage der Viruslast der Proben und der Volkszählungsdaten.

2. Verfahren nach Anspruch 1, wobei das Durchführen von Diagnostik an der Abwasserprobe, um der Probe zugehörige Viruslastinformationen zu ermitteln, ferner das Berücksichtigen einer Verdünnung der Probe umfasst.

3. Verfahren nach Anspruch 1, wobei das Durchführen von Diagnostik an der Abwasserprobe, um der Probe zugehörige Viruslastinformationen zu ermitteln, ferner das Berücksichtigen der Zeit umfasst, die seit der Ausscheidung von in der Probe enthaltenem Stuhl vergangen ist.

4. Verfahren nach Anspruch 1, wobei das Durchführen von Diagnostik an der Abwasserprobe, um der Probe zugehörige Viruslastinformationen zu ermitteln, Folgendes umfasst:
Durchführen einer Echtzeit-Polymerasekettenreaktion; und
Durchführen einer gezielten Sequenzierung auf der Grundlage eines Genoms eines der Viruslast zugehörigen Virus.

5. Verfahren nach Anspruch 1, wobei das Übertragen der Viruslastinformationen auf eine der Region zugehörige Karte Folgendes umfasst:
Erstellen einer Ebene mit den Viruslastinformationen; und
Anzeigen der Ebene mit den Viruslastinformationen auf einer der Karte zugehörigen Ebene.

6. Verfahren nach Anspruch 1, ferner umfassend:
Bestimmen eines Risikos auf der Grundlage der Viruslastinformationen; und
Anzeigen des Risikos.

7. Verfahren nach Anspruch 1, ferner umfassend:
Erlangen demografischer Daten zu bestätigten Fällen, die mit der Viruslast in Zusammenhang stehen;
Identifizieren von Lücken in der Testung auf der Grundlage eines Abgleichs der demografischen Daten mit den Viruslastinformationen; und
Kommunizieren der Lücken in der Testung an einen Benutzer.

8. Verfahren nach Anspruch 7, wobei das Identifizieren von Lücken in der Testung auf der Grundlage eines Abgleichs zwischen den demografischen Daten mit den Viruslastinformationen ferner umfasst:
Bestimmen eines standortbasierten Modells auf der Grundlage von Probeentnahmeinformationen zu diesem Standort, demografischen Daten und Infektionsrate;
Bestimmen einer prognostizierten Infektionsrate für den Standort durch Invertieren des standortbasierten Modells; und
Vergleichen der prognostizierten Infektionsrate mit den demografischen Daten zu bestätigten Fällen, die mit der Viruslast in Zusammenhang stehen.

9. Verfahren nach Anspruch 1, wobei das Durchführen von Diagnostik an der Abwasserprobe, um der Probe zugehörige Viruslastinformationen zu ermitteln, ferner das Verwenden einer an der Probe durchgeführten Echtzeit-Polymerasekettenreaktions-Diagnostik als Gatekeeper umfasst, um die Probe an eine gezielte Sequenzierungsdiagnostik weiterzuleiten.

## Revendications

1. Procédé d'utilisation d'eaux usées pour déterminer l'infection d'une région, le procédé consistant à :
recevoir un échantillon d'eaux usées, dans lequel l'échantillon comprend des informations de localisation ;
réaliser des diagnostics sur l'échantillon d'eaux usées pour déterminer des informations sur la charge virale associées à l'échantillon ;
transposer les informations sur la charge virale sur une carte associée à la région en :
générant une première couche associée aux informations sur la charge virale ;
générant une seconde couche associée aux données du recensement ; et
affichant la première couche et la seconde couche indépendamment l'une de l'autre sur une couche associée à une carte de la région ; et
identifiant les zones à haut risque d'une épidémie potentielle en fonction de la charge virale des échantillons et des données du recensement.

2. Procédé selon la revendication 1, dans lequel la réalisation de diagnostics sur l'échantillon d'eaux usées pour déterminer des informations sur la charge virale associées à l'échantillon comprend en outre la prise en compte d'une dilution de l'échantillon.

3. Procédé selon la revendication 1, dans lequel la réalisation de diagnostics sur l'échantillon d'eaux usées pour déterminer des informations sur la charge virale associées à l'échantillon comprend en outre la prise en compte d'un temps qui s'est écoulé depuis que les selles dans l'échantillon ont été excrétées.

4. Procédé selon la revendication 1, dans lequel la réalisation de diagnostics sur l'échantillon d'eaux usées pour déterminer des informations sur la charge virale associées à l'échantillon comprend :
la réalisation d'une réaction en chaîne par polymérase en temps réel ; et
la réalisation d'un séquençage ciblé basé sur le génome d'un virus associé à la charge virale.

5. Procédé selon la revendication 1, dans lequel la transposition des informations sur la charge virale sur une carte associée à la région comprend :
la génération d'une couche des informations sur la charge virale ; et
l'affichage de la couche des informations sur la charge virale sur une couche associée à la carte.

6. Procédé selon la revendication 1, comprenant en outre :
la détermination d'un risque basé sur les informations sur la charge virale ; et
l'affichage du risque.

7. Procédé selon la revendication 1, comprenant en outre :
l'obtention de données démographiques sur les cas confirmés associés à la charge virale ;
l'identification des lacunes dans les tests en fonction d'une réconciliation entre les données démographiques et les informations sur la charge virale ; et
la communication des lacunes dans les tests à un utilisateur.

8. Procédé selon la revendication 7, dans lequel l'identification des lacunes dans les tests basée sur une réconciliation entre les données démographiques et les informations sur la charge virale comprend en outre :
la détermination d'un modèle basé sur l'emplacement en fonction des informations d'échantillonnage de cet emplacement, de ces données démographiques et de ce taux d'infection ;
la détermination d'un taux d'infection prédit pour l'emplacement en inversant le modèle basé sur l'emplacement ; et
la comparaison du taux d'infection prédit aux données démographiques sur les cas confirmés associés à la charge virale.

9. Procédé selon la revendication 1, dans lequel la réalisation de diagnostics sur l'échantillon d'eaux usées pour déterminer des informations sur la charge virale associées à l'échantillon comprend en outre l'utilisation de diagnostics par réaction en chaîne par polymérase effectués en temps réel sur l'échantillon comme un gardien pour passer l'échantillon sur des diagnostics de séquençage ciblés.
